# EUROPEAN PATENT APPLICATION

(11) **EP 2 703 014 A1**
(43) Date of publication of application: **05.03.2014**
(21) Application number: 12777267.1
(22) Date of filing: 06.04.2012
(51) Int. Cl.: A61L 27/00, C07K 7/08

(54) **SYNTHETIC VITREOUS MATERIAL**

(30) Priority: 27.04.2011 JP 2011099564
(71) Applicant: Menicon Co., Ltd., Nagoya-shi Aichi 460-0006 (JP); Osaka University, Suita-shi, Osaka 565-0871 (JP)
(72) Inventor: UESUGI, Koji, Kasugai-shi Aichi 487-0032 (JP); HAYASHI, Ryuhei, Suita-shi, Osaka 565-0871 (JP); HAYASHIDA, Yasutaka, Suita-shi, Osaka 565-0871 (JP); BABA, Koichi, Suita-shi, Osaka 565-0871 (JP); NISHIDA, Kohji, Suita-shi, Osaka 565-0871 (JP)
(74) Representative: Müller-Boré & Partner Patentanwälte PartG mbB
(86) International application number: PCT/JP2012/059498
(87) International publication number: WO 2012/147497

(57) **Abstract**

Provided is a synthetic vitreous material which is not toxic to the ocular tissue, is capable of maintaining an intraocular tamponade effect for a long term, and is excellent in handleability. A synthetic vitreous material of the present invention includes a self-assembling peptide and a salt, and has an osmotic pressure of 40 mOsm/kg to 200 mOsm/kg. The synthetic vitreous material of the present invention is not toxic to the ocular tissue, is capable of maintaining an intraocular tamponade effect for a long term, and is excellent in handleability.

## Description

### Technical Field

The present invention relates to a synthetic vitreous material.

### Background Art

A vitreous body is a gel-like substance which is formed of collagen and sodium hyaluronate and fills the posterior cavity of an eyeball. The vitreous body has a tamponade effect by which a retina is sustained from the inside of an eyeball, thereby preventing the detachment of the retina. Thus, the vitreous body is an important tissue for an eyeball to function normally. At present, vitrectomy is frequently applied to the vitreoretinal disease which requires operation and treatment, but the removed vitreous body is not replaced by any material because no suitable substitute for a vitreous body is available. Further, it is reported that, after a vitreous body is removed, the effect of intraocular drug administration by injection reduces in comparison to that in the presence of a vitreous body.

Examples of a tamponade material to be used after the removal of a vitreous body include a gas (for example, air or an expansion gas such as sulfur hexafluoride or octafluoropropane), a silicone oil, and a perfluorocarbon liquid. Of those, the silicone oil is frequently used clinically. However, the silicone oil is highly toxic to the ocular tissue, and hence needs to be removed after a certain period of time, thus requiring troublesome handling. In addition, the silicone oil may, for example, emulsify and become cloudy. Further, the perfluorocarbon liquid is used as a temporary tamponade material during operation. However, the perfluorocarbon liquid is also highly toxic to the ocular tissue and is thus removed, at the time of the completion of the operation of an eye, from the eye. When gas tamponade is performed by using air or an expansion gas such as sulfur hexafluoride or octafluoropropane, such a gas is intraocularly absorbed, and hence the effect of the gas tamponade is maintained for only a short period of time from one day to one week (for example, Patent Literature 1). Further, when such gas tamponade is performed during operation of a patient, the patient usually is forcefully required to lie in a prone posture for about one week after the operation. Collagen and hyaluronic acid, and salts thereof are also used as tamponade materials (for example, Patent Literature 2). However, those are derived from organisms and/or microorganisms, thus being expensive, and hence they have not yet been used in clinical applications.

Further, there are proposed, as tamponade materials, a composition using a polyethylene glycol one of the ends of which is modified with a long-chain alkyl group and a polyethylene glycol both ends of which are modified with long-chain alkyl groups (for example, Patent Literature 3). This composition has a high hardness and can be expected to exert a tamponade effect, but it is necessary to use a thicker injection needle (21 gauge) than a usually-used injection needle (25 gauge) in order to improve their handleability. Thus, a larger load is applied to an eyeball and a period of time for which treatment is given may be longer.

### Citation List

### Patent Literature

[PTL 1] JP 6-154263 A
[PTL 2] JP 5-184663 A
[PTL 3] JP 2010-104632 A

### Summary of Invention

### Technical Problem

The present invention has been made to solve the above-mentioned problems, and an object of the present invention is to provide a synthetic vitreous material which is not toxic to the ocular tissue, is capable of maintaining an intraocular tamponade effect for a long term, and is excellent in handleability.

### Solution to Problem

According to the present invention, provided is a synthetic vitreous material. The synthetic vitreous material includes a self-assembling peptide and a salt, and has an osmotic pressure of 40 mOsm/kg to 200 mOsm/kg.

In a preferred embodiment, the above-mentioned synthetic vitreous material includes the above-mentioned self-assembling peptide at 0.01 w/v% to 0.5 w/v%.

In a preferred embodiment, the above-mentioned self-assembling peptide is represented by the following amino acid sequence.
Amino acid sequence: a₁b₁c₁b₂a₂b₃db₄a₃b₅c₂b₆a₄
(In the amino acid sequence, a₁ to a₄ each represent a basic amino acid residue, b₁ to b₆ each represent a non-charged polar amino acid residue and/or a hydrophobic amino acid residue, provided that at least five amino acid residues out of the b₁ to b₆ are each a hydrophobic amino acid residue, c₁ and c₂ each represent an acidic amino acid residue, and d represents a hydrophobic amino acid residue.)

In a preferred embodiment, b₁ to b₆ in the above-mentioned amino acid sequence each independently represent an alanine residue, a valine residue, a leucine residue, or an isoleucine residue.

In a preferred embodiment, d in the above-mentioned amino acid sequence represents an alanine residue, a valine residue, a leucine residue, or an isoleucine residue.

### Advantageous Effects of Invention

The synthetic vitreous material of the present invention is capable of maintaining its intraocular tamponade effect for a long term. Further, the synthetic vitreous material of the present invention has moderate fluidity, thus being excellent in handleability. The synthetic vitreous material of the present invention includes a self-assembling peptide and a salt, and is not toxic to the ocular tissue. Thus, when the synthetic vitreous material of the present invention is used for a patient, it is not necessary to maintain such a prone state after operation as being required after gas tamponade and to perform such a removal operation as being required when a silicone oil or the like is used, and hence the quality of life (QOL) of the patient can be enhanced. Further, the synthetic vitreous material of the present invention is also excellent in drug retention capability, thus being able to prevent the effect of intraocular drug administration from reducing after the removal of a vitreous body.

### Brief Description of the Drawings

FIG. **1a** is a photograph of an anterior eye segment of a domestic rabbit, the photograph being taken one week later after injection of a synthetic vitreous material of the present invention.
FIG. **1b** is a photograph of an ocular fundus of a domestic rabbit, the photograph being taken one week later after injection of the synthetic vitreous material of the present invention.
FIG. **1c** is a photograph of an HE-stained retinal tissue of a domestic rabbit, the photograph being taken one week later after injection of the synthetic vitreous material of the present invention.
FIG. **2a** is a photograph of an anterior eye segment of a domestic rabbit, the photograph being taken one month later after injection of the synthetic vitreous material of the present invention.
FIG. **2b** is a photograph of an ocular fundus of a domestic rabbit, the photograph being taken one month later after injection of the synthetic vitreous material of the present invention.
FIG. **2c** is a photograph of an HE-stained retinal tissue of a domestic rabbit, the photograph being taken one month later after injection of the synthetic vitreous material of the present invention.
FIG. **3a** is a photograph of an anterior eye segment of a domestic rabbit, the photograph being taken three months later after inj ection of the synthetic vitreous material of the present invention.
FIG. **3b** is a photograph of an ocular fundus of a domestic rabbit, the photograph being taken three months later after injection of the synthetic vitreous material of the present invention.
FIG. **3c** is a photograph of an HE-stained retinal tissue of a domestic rabbit, the photograph being taken three months later after injection of the synthetic vitreous material of the present invention.

### Description of Embodiments

### <Definitions of terms>

(1) Herein, the term "self-assembling peptide" refers to a peptide which spontaneously gathers via an interaction between peptide molecules in a solvent. The interaction is not particularly limited, and examples thereof include hydrogen bonding, an interionic interaction, an electrostatic interaction such as the van der Waals force, and a hydrophobic interaction. In one embodiment, the self-assembling peptide is capable of self-assembling into a nanofiber or a gel in a room-temperature aqueous solution (such as an aqueous solution including a peptide at 0.4 w/v%).
(2) Herein, the term "gel" refers to a viscoelastic substance having both viscous property and elastic property.
(3) Herein, the term "hydrophilic amino acid" is meant to include: basic amino acids such as arginine (Arg/R), lysine (Lys/K), and histidine (His/H) ; acidic amino acids such as aspartic acid (Asp/D) and glutamic acid (Glu/E); and non-charged polar amino acids such as tyrosine (Tyr/Y), serine (Ser/S), threonine (Thr/T), asparagine (Asn/N), glutamine (Gln/Q), and cysteine (Cys/C). The alphabets in the parentheses refer to the three letter code and one letter code of an amino acid, respectively.
(4) Herein, the term "hydrophobic amino acid" is meant to include nonpolar amino acids such as alanine (Ala/A), leucine (Leu/L), isoleucine (Ile/I), valine (Val/V), methionine (Met/M), phenylalanine (Phe/F), tryptophan (Trp/W), glycine (Gly/G), and proline (Pro/P). The alphabets in the parentheses refer to the three letter code and one letter code of an amino acid, respectively.

### <Synthetic vitreous material>

A synthetic vitreous material of the present invention includes the self-assembling peptide and the salt. Because the synthetic vitreous material of the present invention includes the self-assembling peptide and the salt, the synthetic vitreous material is capable of maintaining a long-term, intraocular tamponade effect and is excellent in handleability.

The synthetic vitreous material of the present invention has an osmotic pressure of 40 mOsm/kg to 200 mOsm/kg. Because the synthetic vitreous material has an osmotic pressure in the above-mentioned range, the synthetic vitreous material is capable of maintaining a longer-term, intraocular tamponade effect and can be excellent in handleability. When a synthetic vitreous material has an osmotic pressure of more than 200 mOsm/kg, the synthetic vitreous material may have reduced transparency. Note that the osmotic pressure of a synthetic vitreous material can be measured by using the osmometry (osmolality measurement method) in which a cryoscopic method is used and which is in accordance with the Pharmacopeia of Japan.

It is preferred that the pH of the synthetic vitreous material of the present invention be adjusted to a physiological condition (a pH of about 7.4), and the pH can be adjusted by using, for example, any suitable pH adjuster or buffer.

### A. Salt

A salt similar to one contained in a body fluid (for example, an aqueous humor) is preferred as the above-mentioned salt, and any suitable salt can be used. Examples of the above-mentioned salt include ionic salts such as sodium chloride and magnesium chloride. Those salts may be used alone or in combination.

A salt in the form of a salt solution prepared by dissolving any suitable salt in any suitable solvent may be used as the above-mentioned salt. Examples of the solvent used for preparing the salt solution include distilled water. A commercially available salt solution may be used as the salt solution. Specific examples thereof include a physiological saline, a Ringer's solution, and a diluent for an intraocular irrigating solution such as an oxiglutatione solution (for example, a diluent for an oxiglutatione solution packed in BSS PLUS, which is a trade name and manufactured by ALCON JAPAN LTD., and a diluent for an oxiglutatione solution packed in Opeaqua (trademark), which is a trade name and manufactured by Showa Yakuhin Kako Co. , Ltd.). Those salt solutions may be used alone or in combination.

The content ratio of the salt contained in the synthetic vitreous material of the present invention can be adjusted so that the synthetic vitreous material to be obtained has an osmotic pressure of 40 mOsm/kg to 200 mOsm/kg.

### B. Self-assembling peptide

The self-assembling peptide to be used in the present invention has only to be non-toxic to the biological body, in particular, the ocular tissue, and any suitable self-assembling peptide may be used. The synthetic vitreous material of the present invention includes the self-assembling peptide at preferably 0.01 w/v% to 0.5 w/v%, more preferably 0.05 w/v% to 0.4 w/v%. Because the synthetic vitreous material includes the self-assembling peptide at a ratio in any of the above-mentioned ranges, the synthetic vitreous material is capable of maintaining its intraocular tamponade effect for a long period of time and can be excellent in handleability. Attention has also been paid to self-assembling peptides usable as substrates for a drug delivery system. Thus, after the synthetic vitreous material of the present invention is injected intraocularly, the synthetic vitreous material can prevent the effect of intraocular drug administration from reducing. Only one kind of self-assembling peptide may be used, or two or more kinds thereof may be used in combination.

Preferably, the self-assembling peptide to be used in the present invention is represented by the following amino acid sequence.
Amino acid sequence: a₁b₁c₁b₂a₂b₃db₄a₃b₅c₂b₆a₄
(In the amino acid sequence, a₁ to a₄ each represent a basic amino acid residue, b₁ to b₆ each represent a non-charged polar amino acid residue and/or a hydrophobic amino acid residue, provided that at least five amino acid residues out of the b₁ to b₆ are each a hydrophobic amino acid residue, c₁ and c₂ each represent an acidic amino acid residue, and d represents a hydrophobic amino acid residue.)

The self-assembling peptide represented by the above-mentioned amino acid sequence can serve as a synthetic vitreous material which is capable of maintaining its intraocular tamponade effect for a longer period of time. Further, the peptide represented by the above-mentioned amino acid sequence can form, under a physiological condition, a gel excellent in transparency and mechanical strength, and hence the peptide can be suitably used to produce a synthetic vitreous material.

L-amino acids or D-amino acids may be used as amino acids constituting the above-mentioned self-assembling peptide. Further, naturally-occurring amino acids or non-naturally-occurring amino acids may be used. Naturally-occurring amino acids are preferred because the amino acids are available at low prices and can be easily synthesized into a peptide.

In the amino acid sequence, a₁ to a₄ each represent a basic amino acid residue. The basic amino acid is preferably arginine, lysine, or histidine, more preferably arginine or lysine because these amino acids are highly basic. a₁ to a₄ may represent the same amino acid residue, or may represent amino acid residues different from each other.

In the amino acid sequence, b₁ to b₆ each represent a non-charged polar amino acid residue and/or a hydrophobic amino acid residue. Of those, at least five amino acid residues are hydrophobic amino acid residues. The hydrophobic amino acid is preferably alanine, leucine, isoleucine, valine, methionine, phenylalanine, tryptophan, glycine, orproline, and the non-charged polar amino acid is preferably tyrosine, serine, threonine, asparagine, glutamine, or cysteine, because of easy availability of these amino acids.

The b₃ and b₄ each independently are preferably any suitable hydrophobic amino acid residue, more preferably a leucine residue, an alanine residue, a valine residue, or an isoleucine residue, particularly preferably a leucine residue or an alanine residue. When the b₃ and b₄ located at the sixth position and the eighth position, respectively, are hydrophobic amino acid residues in the above-mentioned amino acid sequence, three amino acid residues located at the sixth to eighth positions are continuous hydrophobic amino acid residues. It is presumed that the hydrophobic region formed in the center of the amino acid sequence as mentioned above has a hydrophobic interaction or the like, thus being able to improve the strength of the resultant synthetic vitreous material, and hence the synthetic vitreous material can maintain its intraocular tamponade effect for a long term.

The b₁ to b₆ are all preferably hydrophobic amino acid residues, because the resultantself-assembling peptide can suitably form a β-sheet structure and can be self-assembled. The b₁ to b₆ each independently are more preferably a leucine residue, an alanine residue, a valine residue, or an isoleucine residue, even more preferably a leucine residue or an alanine residue. In a preferred embodiment, 4 or more amino acid residues out of the b₁ to b₆ are leucine residues, particularly preferably, 5 or more amino acid residues out of the b₁ to b₆ are leucine residues, andmost preferably, all amino acid residues are leucine residues. This is because the resultant self-assembling peptide is excellent in solubility in water, thus easily enabling the preparation of a synthetic vitreous material, and because the strength of the synthetic vitreous material can be enhanced, and hence the synthetic vitreous material is capable of maintaining its intraocular tamponade effect for a long term.

In the above-mentioned amino acid sequence, the c₁ and c₂ each represent an acidic amino acid residue. It is preferred that aspartic acid or glutamic acid be used as an acidic amino acid, because these amino acids are easily available. The c₁ and c₂ may be the same amino acid residue or may be different amino acid residues.

In the above-mentioned amino acid sequence, the d represents a hydrophobic amino acid residue. Because the d is a hydrophobic amino acid residue as mentioned above and the self-assembling peptide has a predetermined symmetric structure, it is deemed that a synthetic vitreous material having a better mechanical strength is formed and the material can maintain an intraocular tamponade effect for a long term.

The d is preferably be an alanine residue, a valine residue, a leucine residue, or an isoleucine residue. In this case, length of side chains of amino acids located on the side of a hydrophilic surface in a β-sheet structure formed by the resultant self-assembling peptide can be noncomplementary, but the self-assembling peptide can exert an excellent self-assembling ability, and a synthetic vitreous material which has a better mechanical strength than conventional ones and is capable of maintaining an intraocular tamponade effect for a long term can be obtained.

The sum of the charges, in the neutral region, of the amino acid residues contained in the above-mentionedself-assembling peptide is substantially +2. That is, in the neutral region, plus charges and minus charges which are derived from the side chains of the amino acid residues contained in the above-mentioned self-assembling peptide are not offset to each other. In addition, because both amino acid residues positioned at the N-terminal and C-terminal are basic amino acid residues, it is presumed that, for example, a static repulsive force as well as a static attractive force works between the molecules of the self-assembling peptide to be used in the present invention, a subtle balance is struck between the forces, preventing the occurrence of excessive assembly substantially, and hence the self-assembling peptide can be formed into a stable gel without precipitating in the neutral region, which has a condition close to the physiological condition. Note that, herein, the term "neutral region" refers to a region having a pH of 6 to 8, preferably a pH of 6.5 to 7.5.

The charge of the self-assembling peptide at each pH can be calculated in accordance with, for example, the method of Lehninger (Biochimie, 1979). The method of Lehninger can be performed with, for example, a program available on the web site

(http://www.embl-heidelberg.de/cgi/pi-wrapper.pl) of EMBL WWW Gateway to Isoelectric Point Service.

Preferred specific examples of the self-assembling peptide to be used in the present invention are shown below.
n-RLDLRLALRLDLR-c (SEQ ID NO: 1)
n-RLDLRLLLRLDLR-c (SEQ ID NO: 2)
n-RADLRLALRLDLR-c (SEQ ID NO: 3)
n-RLDLRLALRLDAR-c (SEQ ID NO: 4)
n-RADLRLLLRLDLR-c (SEQ ID NO: 5)
n-RADLRLLLRLDAR-c (SEQ ID NO: 6)
n-RLDLRALLRLDLR-c (SEQ ID NO: 7)
n-RLDLRLLARLDLR-c (SEQ ID NO: 8)

The above-mentioned self-assembling peptide can be produced by any suitable production method. Examples of the production method include a chemical synthetic method such as a solid-phase method, for example, an Fmoc method or a liquid-phase method and a molecular biological method such as gene recombinant expression.

The above-mentioned self-assembling peptide may be a self-assembling peptide with any suitable modification (hereinafter referred to as a modified peptide). The modified peptide is a peptide which has a self-assembling ability and is prepared by applying any suitable modification to the above-mentioned self-assembling peptide to such an extent that the modified peptide is not toxic to the biological body, in particular, the ocular tissue. The site to which the modification is applied may be the N-terminal amino group of the above-mentioned self-assembling peptide or may be the C-terminal carboxyl group thereof. Alternatively, both the sites may be modified.

Any suitable modification can be selected as the above-mentioned modification as long as the resultant modified peptide has a self-assembling ability and is not toxic to the biological body, in particular, the ocular tissue. Examples of the modification include: the introduction of a protective group such as acetylation of an N-terminal or amidation of a C-terminal; the introduction of a functional group such as alkylation, esterification, or halogenation; hydrogenation; the introduction of a saccharide compound such as a monosaccharide, a disaccharide, an oligosaccharide, or a polysaccharide; the introduction of a lipid compound such as a fatty acid, a phospholipid, or a glycolipid; the introduction of an amino acid or a protein; the introduction of DNA; and the introduction of, for example, other compounds each having bioactivity. When an amino acid or a protein is introduced, the peptide obtained after the introduction is a peptide in which any suitable amino acid is added to the N-terminal and/or C-terminal of the above-mentioned self-assembling peptide. Herein, the added peptide is also included in the modified peptide. Only one kind of modification may be applied, or two or more kinds thereof may be applied in combination. For example, the followingmaybe adopted: a desired amino acid is introduced into the C-terminal of the above-mentioned self-assembling peptide, yielding an added peptide, the N-terminal of the added peptide is acetylated, and the C-terminal thereof is amidated.

The above-mentioned added peptide (modified peptide) does not have, as a whole, features of the above-mentioned self-assembling peptide in some cases. Specific examples of such cases include the case that, owing to the addition of any suitable amino acid, the sequence toward the N-terminal direction and the sequence toward the C-terminal direction are asymmetric with respect to a hydrophobic amino acid residue at position 7 in the center, and the case that the added peptide has hydrophobic amino acids and hydrophilic amino acids at the same ratio. Even in such case, because the above-mentioned self-assembling peptide has an extremely excellent self-assembling ability, the added peptide obtained by adding any appropriate amino acid to the self-assembling peptide can also provide a synthetic vitreous material which is excellent in mechanical strength and is capable of maintaining an intraocular tamponade effect for a long term.

In the case of introducing some amino acids or proteins, the number of the amino acid residues constituting the modified peptide obtained after the introduction is preferably 14 to 200, more preferably 14 to 100, evenmore preferably 14 to 50, particularly preferably 14 to 30, most preferably 14 to 20. When the number of the amino acid residues is more than 200, the self-assembling ability of the above-mentioned self-assembling peptide is sometimes impaired.

The kinds and positions of the amino acids to be introduced can be properly set depending on the applications of the resultant modified peptide and the like. It is preferred that amino acids be introduced so that a hydrophobic amino acid and a hydrophilic amino acid are positioned alternately, starting from the arginine residues/residue (hydrophilic amino acids/acid) located at the N-terminal and/or C-terminal of the above-mentioned self-assembling peptide.

The above-mentioned modification can be performed by any suitable method depending on the kind thereof and the like.

### C. Additive

The synthetic vitreous material of the present invention may include any suitable additive in addition to the above-mentioned self-assembling peptide and salt. Examples of the additive include any suitable chemicals, for example, low-molecular-weight compounds, nucleic acids such as DNA and RNA, and antibodies such as Lucentis, Avastin, and Macugen.

### D. Production method for synthetic vitreous material

The synthetic vitreous material of the present invention can beproducedbyanysuitablemethod. For example, the above-mentioned self-assembling peptide is dissolved in distilled water so as to prepare a peptide aqueous solution having a desired concentration, and the peptide aqueous solution, the above-mentioned salt, any suitable additive, if required, and a solvent are agitated and mixed by using any suitable agitation means, thereby being able to yield a synthetic vitreous material. Another example of the suitable method is as follows: the above-mentioned peptide aqueous solution, the above-mentioned salt solution, and any suitable additive, if required, are agitated and mixed by using any suitable agitation means, thereby being able to yield a synthetic vitreous material.

### E. Use method for synthetic vitreous material

The synthetic vitreous material of the present invention can be injected into an eyeball by using any suitable means. For example, the synthetic vitreous material of the present invention is filled in a syringe, followed by sterilization treatment, and the synthetic vitreous material can be injected into an eyeball by using an injector. The synthetic vitreous material of the present invention is excellent in handleability, and hence it is possible to inject the synthetic vitreous material easily into an eyeball even when an injection needle which is thinner than a 25-gauge injection needle usually used for injection into an eyeball is used.

### Examples

Hereinafter, the present invention is specifically described on the basis of examples, but the present invention is not limited by these examples. Note that the osmotic pressure of each synthetic vitreous material was measured as described below.

### (Measurement of osmotic pressure)

Each synthetic vitreous material was diluted by using distilled water (trade name: Official Otsuka Distilled Water, manufactured by Otsuka Pharmaceutical Factory, Inc.) until a solution state was achieved. Next, the osmotic pressure of each diluted synthetic vitreous material was measured by using an osmometer (trade name: Osmometer 3900, manufactured by Advanced Instruments, Inc.) in accordance with the osmometry (osmolality measurement method) described in the Pharmacopeia of Japan. The value of each osmotic pressure obtained was proportionally calculated on the basis of each dilution rate, thereby determining the osmotic pressure of each synthetic vitreous material.

### (Example 1)

A self-assembling peptide (trade name: PanaceaGel SPG-178, manufactured by Menicon Co., Ltd., 1 w/v%) was mixed with distilled water (trade name: Official Otsuka Distilled Water, manufactured by Otsuka Pharmaceutical Factory, Inc.), yielding a peptide aqueous solution having a peptide concentration of 0.15 w/v%. The peptide aqueous solution yielded and a salt solution 1 (a diluent for an oxiglutatione solution with an osmotic pressure of 308 mOsm/kg packed in Opeaqua (trademark), manufactured by Showa Yakuhin Kako Co., Ltd.) were mixed at a volume ratio of 2:1, yielding a synthetic vitreous material 1. Table 1 shows the concentration of the self-assembling peptide, ratio of the salt solution, and osmotic pressure in the synthetic vitreous material obtained.

### (Example 2)

A synthetic vitreous material 2 was obtained in the same manner as that in Example 1, except that a salt solution 2 (a diluent for an oxiglutatione solution with an osmotic pressure of 308 mOsm/kg packed in BSS PLUS (trademark), which was a trade name and was manufactured by ALCON JAPAN LTD.) was used in place of the salt solution 1. Table 1 shows the concentration of the self-assembling peptide, ratio of the salt solution, and osmotic pressure in the synthetic vitreous material obtained.

### (Example 3)

A synthetic vitreous material 3 was obtained in the same manner as that in Example 2, except that the peptide concentration of the resultant peptide aqueous solution was set to 0.45 w/v%. Table 1 shows the concentration of the self-assembling peptide, ratio of the salt solution, and osmotic pressure in the synthetic vitreous material obtained.

### (Example 4)

A synthetic vitreous material 4 was obtained in the same manner as that in Example 1, except that the peptide concentration of the resultant peptide aqueous solution was set to 0.075 w/v%. Table 1 shows the concentration of the self-assembling peptide, ratio of the salt solution, and osmotic pressure in the synthetic vitreous material obtained.

### (Example 5)

A synthetic vitreous material 5 was obtained in the same manner as that in Example 2, except that the peptide concentration of the resultant peptide aqueous solution was set to 0.25 w/v% and the mixing ratio of the peptide aqueous solution to the salt solution 2 was set to a volume ratio of 2:3. Table 1 shows the concentration of the self-assembling peptide, ratio of the salt solution, and osmotic pressure in the synthetic vitreous material obtained.

### (Comparative Example 1)

A peptide aqueous solution having a peptide concentration of 1 w/v% was prepared in the same manner as that in Example 1. Distilled water (trade name: Official Otsuka Distilled Water, manufactured by Otsuka Pharmaceutical Factory, Inc.) was further added to the peptide aqueous solution so that the concentration of the self-assembling peptide came down to 0.1 w/v%, yielding a synthetic vitreous material C1. Table 1 shows the concentration of the self-assembling peptide, ratio of the salt solution, and osmotic pressure in the synthetic vitreous material obtained.

### (Comparative Example 2)

A synthetic vitreous material C2 was obtained in the same manner as that in Example 1, except that the peptide concentration of the resultant peptide aqueous solution was set to 1 w/v% and the mixing ratio of the peptide aqueous solution to the salt solution was set to a volume ratio of 3:7. Table 1 shows the concentration of the self-assembling peptide, ratio of the salt solution, and osmotic pressure in the synthetic vitreous material obtained.

### (Comparative Example 3)

A synthetic vitreous material C3 was obtained in the same manner as that in Example 1, except that the peptide concentration of the resultant peptide aqueous solution was set to 0.2 w/v% and the mixing ratio of the peptide aqueous solution to the salt solution was set to a volume ratio of 9:1. Table 1 shows the concentration of the self-assembling peptide, ratio of the salt solution, and osmotic pressure in the synthetic vitreous material obtained.

**[Table 1]**

| | Peptide concentration in self-assembling peptide aqueous solution (w/v%) | Salt solution | Synthetic vitreous material | | |
|---|---|---|---|---|---|
| | | | Concentration of self-assembling peptide (w/v%) | Ratio of salt solution (v/v%) | Osmotic pressure (mOsm/kg) |
| Example 1 | 0.15 | Salt solution 1 | 0.1 | 33 | 103 |
| Example 2 | 0.15 | Salt solution 2 | 0.1 | 33 | 103 |
| Example 3 | 0.45 | Salt solution 2 | 0.3 | 33 | 103 |
| Example 4 | 0.075 | Salt solution 1 | 0.05 | 33 | 103 |
| Example 5 | 0.25 | Salt solution 2 | 0.1 | 60 | 185 |
| Comparative Example 1 | 1 | - | 0.1 | - | 0 |
| Comparative Example 2 | 1 | Salt solution 1 | 0.3 | 70 | 216 |
| Comparative Example 3 | 0.2 | Salt solution 1 | 0.18 | 10 | 31 |

### (Evaluation)

Each of the synthetic vitreous materials obtained in Examples 1 to 5 and Comparative Examples 1 to 3 was heated to 37 °C by using an incubator (trade name: CO₂ Incubator, manufactured by SANYO Electric Co., Ltd.), and the following evaluations were performed.

### <Tamponade effect, physical properties, and transparency>

The tamponade effect, physical properties, and transparency of each of the heated synthetic vitreous materials were visually confirmed and evaluated. The tamponade effect was evaluated as described below. Table 2 shows the results of the evaluation.
Tamponade effect ⊚: Having a high tamponade effect.
○: Having a tamponade effect.
×: Having no tamponade effect.

### <Handleability>

Each of the heated synthetic vitreous materials was filled in an injector (injection needle: 26 gauge), and the handleability thereof was evaluated on the basis of the feeling (handling) sensed at the time of shooting out each of the materials. Table 2 shows the results of the evaluation.

**[Table 2]**

| | Tamponade effect | Property and state of synthetic vitreous material | Transparency | Handleability |
|---|---|---|---|---|
| Example 1 | ○ | State of gel having high liquidity | ○ | Easy |
| Example 2 | ○ | State of gel having high liquidity | ○ | Easy |
| Example 3 | ⊚ | State of gel having moderate liquidity | ○ | Easy |
| Example 4 | ○ | State of gel having high liquidity | ○ | Easy |
| Example 5 | ○ | State of fragile agglomerate having low liquidity | ○ | Easy |
| Comparative Example 1 | × | Liquid state | ○ | Easy |
| Comparative Example 2 | ⊚ | State of gel having moderate liquidity | × | Easy |
| Comparative Example 3 | × | Liquid state | ○ | Easy |

Each of the synthetic vitreous materials obtained in Examples 1 to 5 had a tamponade effect, was excellent inhandleability, and had high transparency, and hence the materials can be suitably used as synthetic vitreous materials. A 25-gauge injection needle is usually used for injection into an eyeball. The synthetic vitreous material of the present invention was excellent in handleability even when a 26-gauge injection needle thinner than the usual one was used.

The synthetic vitreous materials obtained in Comparative Example 1 and Comparative Example 3 were in liquid states, and hence no satisfactory tamponade effect was exerted. On the other hand, the synthetic vitreous material obtained in Comparative Example 2 was excellent in tamponade effect and handleability but inferior in transparency, and hence the material was not suitable for being used as a synthetic vitreous material.

### (Test example) Injection test into eyeball of domestic rabbit

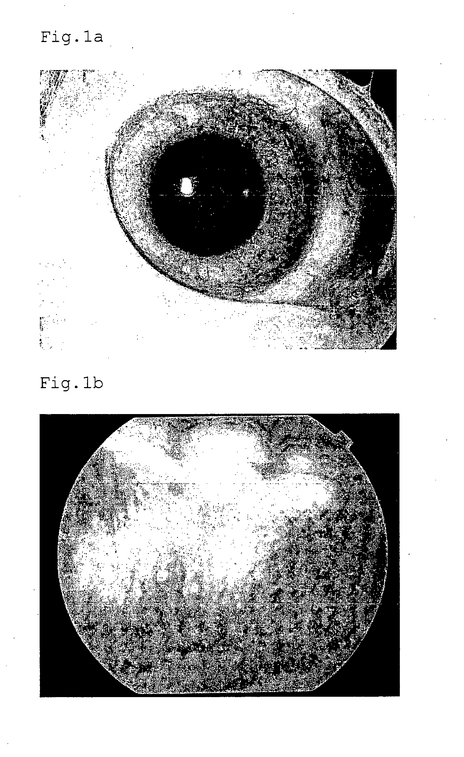

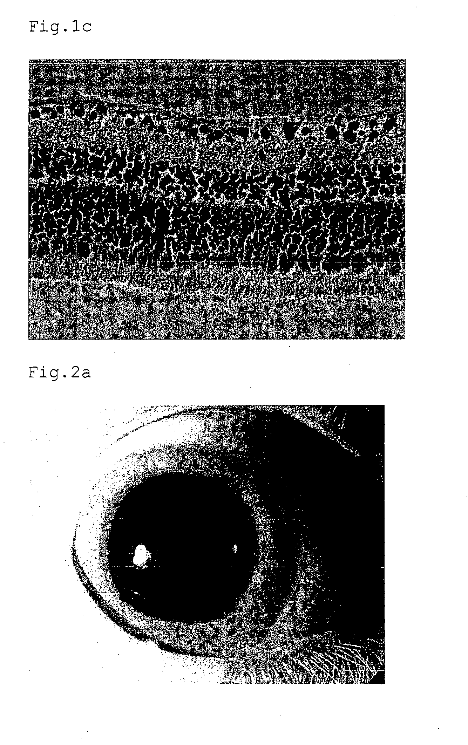

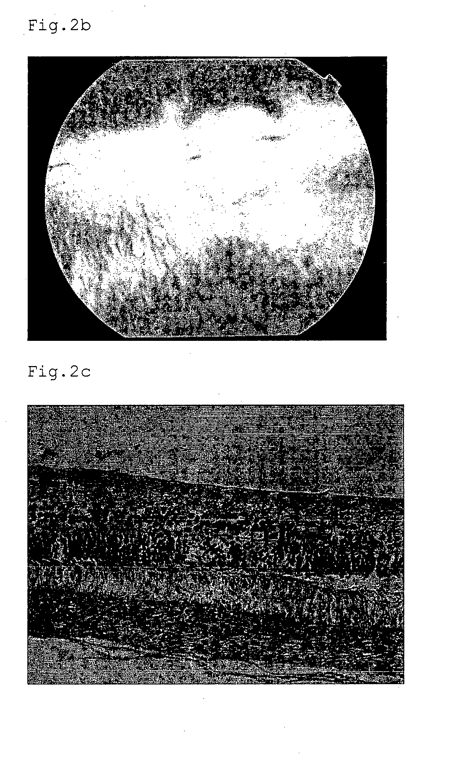

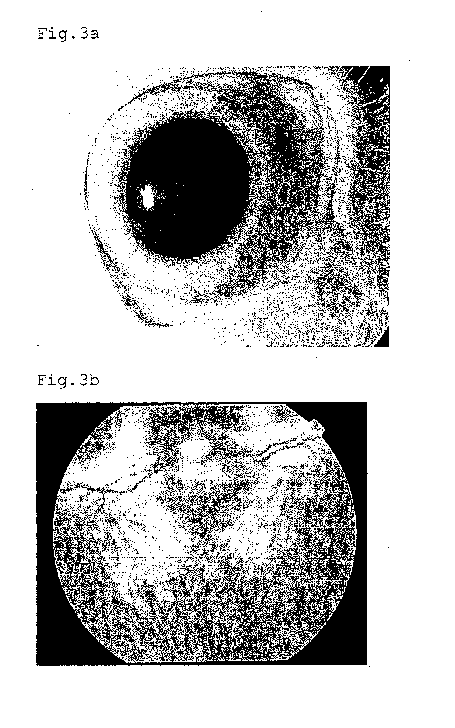

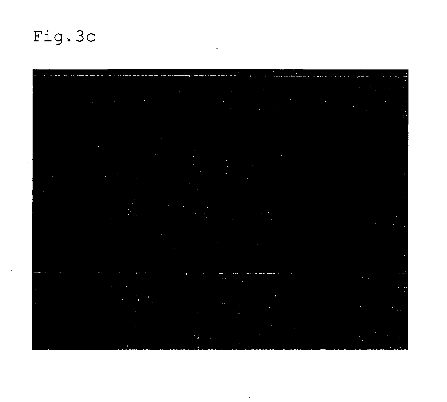

21 white domestic rabbits each having a body weight of 2 kg were used as specimens, and ketamine and xylazine were intramuscularly injected thereinto at 15 mg/kg and 10 mg/kg, respectively, thereby deeply anesthetizing the rabbits. Disappearance of corneal reflex and disappearance of reaction to pain stimulation were confirmed. Subsequently, vitrectomy was applied to one eyeball of each specimen, and then the synthetic vitreous material 2 obtained in Example 2 was injected into each eyeball to complete the operation. A three-port system (25 G), which was widely used in human clinical medicine, was used as a method for the operation. One day later after the operation, three days later, one week later, two weeks later, three weeks later, one month later, and three months later, each eyeball into which the synthetic vitreous material had been injected was observed with a slit-lamp microscope and a fundus microscope, followed by the measurement of electroretinograms. On each observation day, each eyeball into which the synthetic vitreous material had been injected was extirpated from three domestic rabbit specimens, followed by HE staining, and the state of each retina was observed. FIG. **1a** shows a photograph of an anterior eye segment, FIG. **1b** shows a photograph of an ocular fundus, and FIG. **1c** shows a photograph of an HE-stained retinal tissue, the photographs being taken one week later after the operation. Similarly, FIG. **2a** shows a photograph of an anterior eye segment, FIG. **2b** shows a photograph of an ocular fundus, and FIG. **2c** shows a photograph of an HE-stained retinal tissue, the photographs being taken one month later after the operation. FIG. **3a** shows a photograph of an anterior eye segment, FIG. **3b** shows a photograph of an ocular fundus, and FIG. **3c** shows a photograph of an HE-stained retinal tissue, the photographs being taken three months later after the operation.

On all observation days, all crystalline lenses and all synthetic vitreous materials were not cloudy, and all ocular fundi were able to be observed. Even one month later after the operation, the onset of cataract was not observed and the synthetic vitreous material was found to be not toxic to the retinal tissue. In addition, even one month later after the operation, the synthetic vitreous material remained in each eyeball and a good tamponade effect thereof was maintained. Further, the synthetic vitreous material itself remained without hardening and becoming cloudy. Even three months later after the operation, the onset of cataract was not observed and the synthetic vitreous material was found to be not toxic to the retinal tissue. In addition, the synthetic vitreous material neither hardened nor became cloudy and maintained its good tamponade effect. As mentioned above, the synthetic vitreous material of the present invention had excellent handleability, and even after as long a period of time as three months passed, the synthetic vitreous material was not toxic to the ocular tissue and was able to maintain its good intraocular tamponade effect.

### Industrial Applicability

The synthetic vitreous material of the present invention can be suitably used as a tamponade material which is used after vitrectomy.

### Sequence Listing Free Text

SEQ ID NO. 1 is a self-assembling peptide to be used in the present invention.
SEQ ID NO. 2 is a self-assembling peptide to be used in the present invention.
SEQ ID NO. 3 is a self-assembling peptide to be used in the present invention.
SEQ ID NO. 4 is a self-assembling peptide to be used in the present invention.
SEQ ID NO. 5 is a self-assembling peptide to be used in the present invention.
SEQ ID NO. 6 is a self-assembling peptide to be used in the present invention.
SEQ ID NO. 7 is a self-assembling peptide to be used in the present invention.
SEQ ID NO. 8 is a self-assembling peptide to be used in the present invention.

## Claims

1. A synthetic vitreous material, comprising a self-assembling peptide and a salt, wherein the synthetic vitreous material has an osmotic pressure of 40 mOsm/kg to 200 mOsm/kg.

2. A synthetic vitreous material according to claim 1, wherein the synthetic vitreous material comprises the self-assembling peptide at 0.01 w/v% to 0.5 w/v%.

3. A synthetic vitreous material according to claim 1 or 2, wherein the self-assembling peptide is represented by the following amino acid sequence:
amino acid sequence: a₁b₁c₁b₂a₂b₃db₄a₃b₅c₂b₆a₄
where a₁ to a₄ each represent a basic amino acid residue, b₁ to b₆ each represent an uncharged polar amino acid residue and/or a hydrophobic amino acid residue, provided that at least five amino acid residues out of the b₁ to b₆ are each a hydrophobic amino acid residue, c₁ and c₂ each represent an acidic amino acid residue, and d represents a hydrophobic amino acid residue.

4. A synthetic vitreous material according to claim 3, wherein b₁ to b₆ in the amino acid sequence each independently represent an alanine residue, a valine residue, a leucine residue, or an isoleucine residue.

5. A synthetic vitreous material according to claim 3 or 4, wherein d in the amino acid sequence represents an alanine residue, a valine residue, a leucine residue, or an isoleucine residue.


SEQUENCE LISTING

<110>  Menicon Co. Ltd.
       Osaka University

<120>  Artificial vitreous material

<130>  MNC11087PCT

<150>  JP2011-099564
<151>  2011-04-27

<160>  8

<170>  PatentIn version 3.1

<210>  1
<211>  13
<212>  PRT
<213>  Artificial

<220>
<223>  self-assembling peptide

<400>  1

Arg Leu Asp Leu Arg Leu Ala Leu Arg Leu Asp Leu Arg
1               5                   10


<210>  2
<211>  13
<212>  PRT
<213>  Artificial

<220>
<223>  self-assembling peptide

<400>  2

Arg Leu Asp Leu Arg Leu Leu Leu Arg Leu Asp Leu Arg
1               5                   10


<210>  3
<211>  13
<212>  PRT
<213>  Artificial

<220>
<223>  self-assembling peptide

<400>  3

Arg Ala Asp Leu Arg Leu Ala Leu Arg Leu Asp Leu Arg
1               5                   10


<210>  4
<211>  13
<212>  PRT
<213>  Artificial

<220>
<223>  self-assembling peptide

<400>  4

Arg Leu Asp Leu Arg Leu Ala Leu Arg Leu Asp Ala Arg
1               5                   10


<210>  5
<211>  13
<212>  PRT
<213>  Artificial

<220>
<223>  self-assembling peptide

<400>  5

Arg Ala Asp Leu Arg Leu Leu Leu Arg Leu Asp Leu Arg
1               5                   10


<210>  6
<211>  13
<212>  PRT
<213>  Artificial

<220>
<223>  self-assembling peptide

<400>  6

Arg Ala Asp Leu Arg Leu Leu Leu Arg Leu Asp Ala Arg
1               5                   10


<210>  7
<211>  13
<212>  PRT
<213>  Artificial

<220>
<223>  self-assembling peptide

<400>  7

Arg Leu Asp Leu Arg Ala Leu Leu Arg Leu Asp Leu Arg
1               5                   10


<210>  8
<211>  13
<212>  PRT
<213>  Artificial

<220>
<223>  self-assembling peptide

<400>  8

Arg Leu Asp Leu Arg Leu Leu Ala Arg Leu Asp Leu Arg
1               5                   10